# EUROPEAN PATENT SPECIFICATION

(11) **EP 3 269 321 B1**
(45) Date of publication and mention of the grant of the patent: **01.07.2020**
(21) Application number: 17183872.5
(22) Date of filing: 14.03.2014
(51) Int. Cl.: A61B 17/88, A61B 17/70, A61B 17/86

(54) **INSTRUMENT FOR HOLDING AND INSERTING A BONE ANCHOR**
INSTRUMENT ZUM HALTEN UND EINFÜHREN EINES KNOCHENANKERS
INSTRUMENT POUR TENIR ET INSÉRER UN ANCRAGE OSSEUX

(43) Date of publication of application: 17.01.2018
(62) Divisional of application: 14160107.0
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Biedermann, Timo, 78647 Trossingen (DE)
(74) Representative: Prüfer & Partner mbB Patentanwälte · Rechtsanwälte

(56) References cited:
- US-A- 5 667 513
- US-B1- 6 189 422

## Description

The invention relates to an instrument for holding and inserting a bone anchor into the bone. The instrument comprises a holding member including at least two arms that are configured to hold a head of the bone anchor between them and a displacement member that acts onto the holding member so that the holding member can assume a first configuration in which it is configured to receive the head and a second configuration in which it holds an inserted head. Further, the instrument comprises a drive shaft that is configured to engage the head of the bone anchor. A particular application of the instrument is the use with a polyaxial screw of the bottom loading type that comprises a bone anchor and a receiving part to receive a rod to connect the rod to the bone anchor. In this application, the bone anchor is first inserted into the bone using the instrument and the receiving part is mounted thereafter *in situ* onto the bone anchor.

An instrument for holding and inserting of bone screws, in particular of pedicle screws, is known from DE 20 2005 012 781 U1. The instrument comprises a holding member with at least two gripping arms for a bone screw and a displacement member that is movable relative to the gripping arms. By means of the displacement member the gripping arms can assume a configuration which allows the insertion of the bone anchor and a configuration in which the bone anchor is held between the arms. The gripping arms comprise projections that engage recesses on the bone screw in order to allow the screwing-in of the bone screw via the gripping arms.

US 2012/0296171 A1 describes an inserter for a bone anchor comprising a driver for engaging the head of a bone screw and a receiver member with a spherical inner surface portion that becomes engaged with the head and/or the neck of the bone anchor.

US 6,189,422 B1 discloses screwdriver for introducing and screwing a screw into the human or animal body. The screwdriver has a handle and a shaft whose front end has a blade that can be brought into engagement with a screw head for screwing the screw. The screwdriver furthermore has a retention device for retaining the screw on the blade. The retention device has an inner tubular shaft which surrounds the shaft and has at the distal end a clamping gripper preloaded into its open position, and an outer tubular shaft surrounding the inner tubular shaft. An actuation device for the inner tubular shaft and outer tubular shaft is movable from a first into a second position, as a result of which the opened clamping gripper is displaced over the blade out of a pulled-back position. The actuation device is movable out of the second into a third position, as a result of which the outer tubular shaft is displaced relative to the inner tubular shaft over the clamping gripper in order to close the latter.

It is the object of the invention to provide an instrument for holding and inserting a bone anchor that provides a safe and robust connection between the bone anchor and the instrument and an automatic alignment of the bone screw and the instrument.

The object is solved by a system comprising a bone anchor and an instrument for holding and inserting the bone anchor according to claim 1. Further developments are given in the dependent claims. The instrument includes a holding member with a seat for the head of a bone anchor, a displacement member for acting onto the holding member such that the holding member can assume a first configuration in which it is configured to permit the head to enter the seat and a second configuration in which it is configured to hold the inserted head, and a drive shaft that is configured to engage the head of the bone anchor. A center of the seat is offset from a center of the head in such a manner that the head is pressed against an abutment surface. Thereby, a play between an engagement portion of the drive shaft and the head is eliminated. This allows a precise insertion of the bone anchor without experiencing a play between the drive shaft and the bone anchor. Also, by means of this design, the connection between the instrument and the bone anchor is robust.

The head of the bone anchor may be held in the seat in a form-fit or positive-fit manner. Furthermore, the drive shaft of the instrument may comprise an engagement portion with a structure that engages a corresponding engagement portion of the head of the bone anchor in a form-fit manner. Therefore, the bone anchor is automatically aligned correctly with the drive shaft. The size and position of the seat may be designed such that a lower end of the holding member acts as an abutment for the bone surface. Hence, when the bone anchor is inserted with the instrument, the free lower end of the holding member abuts against the bone surface and indicates that a correct insertion of the bone anchor has been reached that allows to mount the receiving part thereafter *in situ.*

Further features and advantages of the invention will become apparent from the description of embodiments by means of the accompanying drawings. In the drawings:
- Fig. 1: shows a perspective exploded view of the instrument according to an embodiment.
- Fig. 2: shows a perspective view of the instrument of Fig. 1 in an assembled state in a first configuration wherein it is configured to receive a head of a bone anchor.
- Fig. 3: shows a perspective view of the instrument of Fig. 3 in a second configuration wherein the head of the bone anchor is received and clamped by the instrument.
- Fig. 4: shows a cross-sectional view of a main portion of the instrument, the cross-section taken in a plane containing the longitudinal axis of the instrument.
- Fig. 5: shows an enlarged cross-sectional view of an upper portion of the instrument of Fig. 4.
- Fig. 6: shows an enlarged cross-sectional view of a lower portion of the instrument of Fig. 4.
- Fig. 7: shows a perspective view of the drive shaft of the instrument of Figs. 1 to 6.
- Fig. 8: shows an enlarged view of portions of the drive shaft of Fig. 7.
- Fig. 9: shows a perspective view from the front end side of a holding member of the instrument of Figs. 1 to 6.
- Fig. 10: shows a perspective view from the rear end side of the holding member shown in Fig. 9.
- Fig. 11: shows a top view onto the rear end of the holding member of Figs. 9 and 10.
- Fig. 12: shows a cross-sectional view of the holding member of Figs. 9 to 10 along line A-A in Fig. 11.
- Fig. 13: shows a perspective view from the rear end side of a displacement member of the instrument according to Figs. 1 to 6.
- Fig. 14: shows a perspective view from the front end side onto the displacement member of Fig. 13.
- Fig. 15: shows a top view onto the rear end of the displacement member of Figs. 13 and 14.
- Fig. 16: shows a cross-sectional view of the displacement member of Figs. 13 and 14.
- Fig. 17: shows a perspective view from the rear end side of an actuator of the instrument according to Figs. 1 to 6.
- Fig. 18: shows a perspective view from the front end side onto the actuator of Fig. 17.
- Fig. 19: shows a top view from the rear end of the actuator of Figs. 17 and 18.
- Fig. 20: shows a cross-sectional view of the actuator of Figs. 17 and 18 along line C-C in Fig. 19.
- Fig. 21: shows a cross-sectional view of a portion of the instrument in a first step of inserting a head of the bone anchor when the instrument is in the first configuration.
- Fig. 22: shows an enlarged cross-sectional view of a portion of the instrument in the first configuration with the inserted head of the bone anchor.
- Fig. 23a: shows a cross-sectional view of the instrument in the second configuration with the inserted head of the bone anchor.
- Fig. 23b: shows an enlarged cross-sectional view of a detail of Fig. 23a.
- Figs. 24 to 26: show schematic enlarged cross-sectional views of a lower portion of the instrument with inserted head.

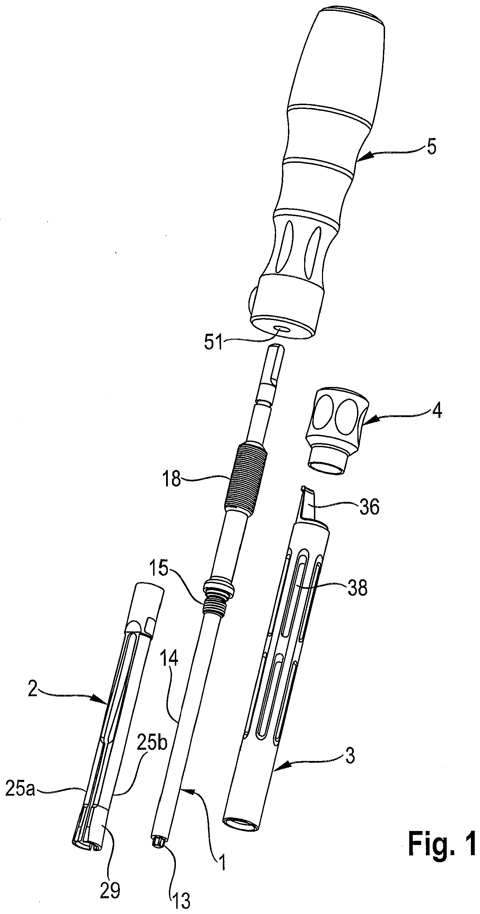

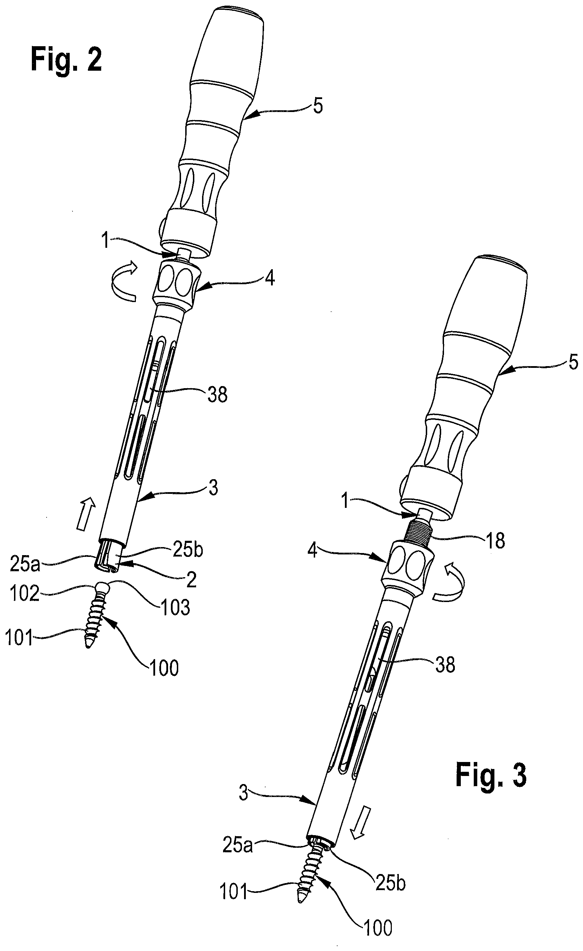

Referring to Figs. 1 to 3, an instrument for holding and inserting a bone anchor into the bone according to an embodiment includes a drive shaft 1 that is configured to engage a head portion of the bone anchor and to transmit torque to the bone anchor, a holding member 2 for receiving and holding the head of the bone anchor, and a displacement member 3 that is configured to act onto the holding member 2. The holding member can assume a first configuration as depicted in Fig. 2 wherein the head of the bone anchor can be inserted into the holding member, and a second configuration as depicted in Fig. 3, wherein the head of the bone anchor is firmly held in the holding member 2. The instrument further includes an actuator 4 that cooperates with the drive shaft 1 and the displacement member 3 such as to move the displacement member 3 into a first and second position corresponding to the first and second configuration of the holding member 2. Finally, the instrument includes a handle 5 that is connectable to the drive shaft 1.

Referring more in detail to Figs. 2 and 3, a bone anchor 100 in this embodiment comprises a threaded shank 101 and a head 102 at one end of the threaded shank 101. The head 102 typically has an surface portion that is spherically-shaped and a substantially flat free end surface 103. The spherical surface portion may include a region of the greatest diameter of the sphere. Such bone anchors are commonly used in polyaxial bone screws that serve for stabilization of the spinal column by means of stabilizing rods. The polyaxial bone screws typically have a receiving part (not shown) that is configured to pivotably receive the bone anchor 100 and to receive the spinal stabilization rod (not shown) to couple the rod to the bone anchor. The instrument is particularly applicable to bottom loading type polyaxial bone screws wherein the head 102 of the bone anchor 100 is inserted into the receiving part from the bottom end thereof, i.e. from the end that faces the bone surface.

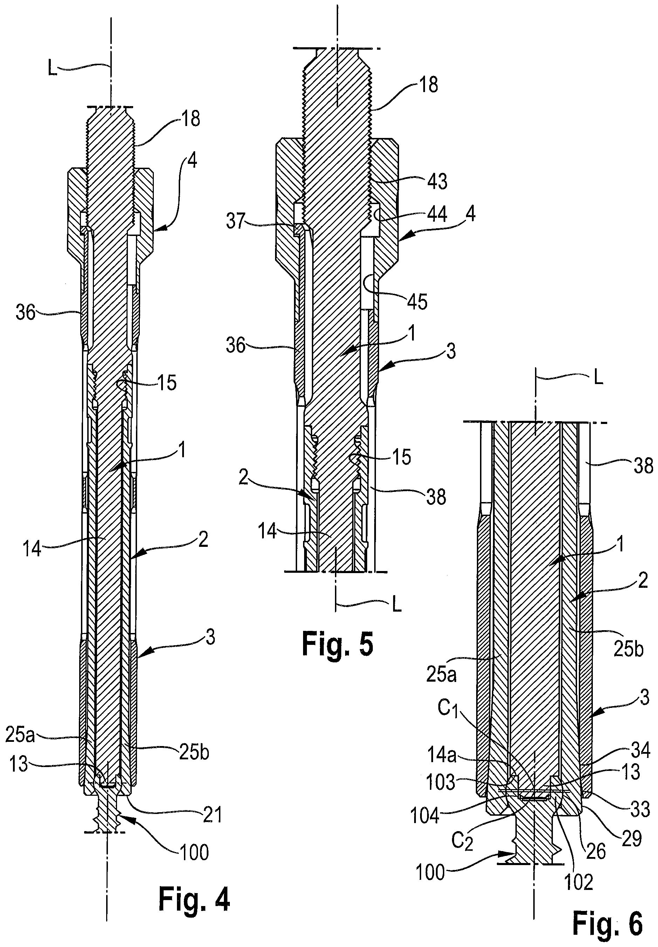

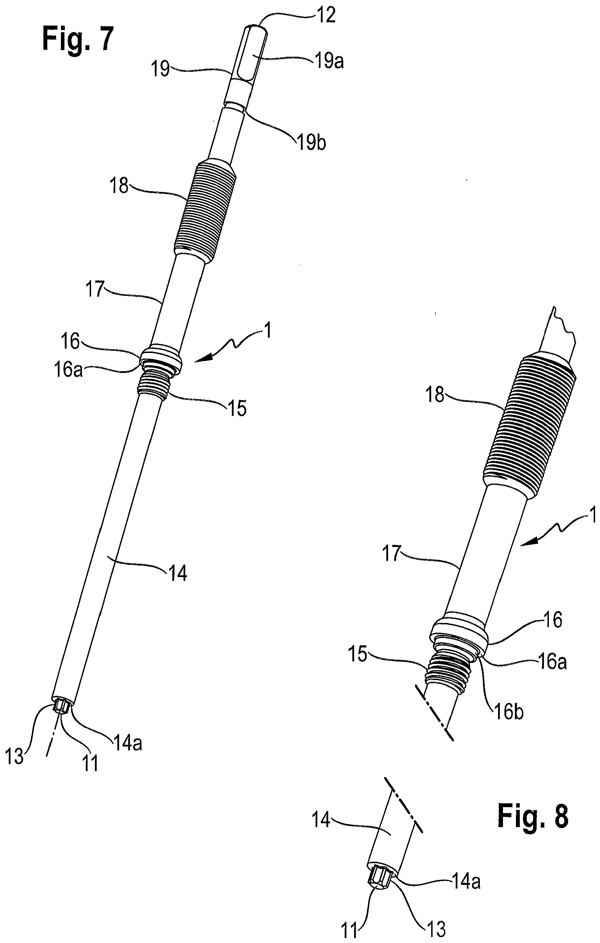

With reference to Figs. 4 to 8, the drive shaft 1 may be a monolithic substantially rod-shaped member that comprises a first end 11, that is in use the proximal end, and an opposite second end 12, that is in use a distal end. At the first end 11 there is an engagement projection 13 is provided that is configured to engage a corresponding recess in the head of head 102 of the bone anchor to transmit torque to the head 102. The engagement projection 13 has a torx-shape. However, any other shape is possible that allows to connect the engagement projection 13 in a form-fit or positive-fit manner to the head 102 of the bone anchor 100. Following the engagement projection 13, the drive shaft 1 comprises a first shaft portion 14 with a first diameter that is greater than that of the engagement projection 13. At the front end of the first shaft portion 14 an abutment surface 14a is formed that extends circumferentially around the engagement projection 13 and that is substantially flat.

At the side opposite to the engagement projection 13, the first shaft portion 14 comprises a threaded section 15 with an outer thread that serves for cooperating with a corresponding inner thread of the holding member 2 and that allows to mount in the holding member 2 onto the shaft portion 14. At a distance from the threaded section 15 there is a section 16 with a larger outer diameter so that an annular abutment surface 16a facing towards the first end 11 is provided. This abutment surface 16a functions as a stop for the holding member 2 when the holding member is mounted to the first shaft portion 14. An intermediate portion 16b with a slightly smaller diameter than the portion 16 may be present to improve the stability and or the alignment of the connection between the holding member 2 and the drive shaft 1.

Between the distal end 12 and the larger diameter section 16 there is a second shaft portion 17 that comprises approximately at the middle section a portion 18 with a greater outer diameter. The portion 18 is threaded and cooperates with the actuator 4. Preferably, the thread of the portion 18 is a fine pitch thread and more particularly, it may be a multi-start fine pitch thread, such as a double-start fine pitch thread. This allows to obtain a greater displacement in the axial direction of the actuator 4 when the actuator 4 is screwed back and forth along the threaded section 18. Hence, the displacement of the displacement member 3 can be achieved with less turns or in shorter time. An end section 19 adjacent to the second end 12 comprises one or more longitudinal flats 19a compared to a single-start thread to provide a form-fit connection to the handle 5 such that the handle 5 can not rotate relative to the drive shaft 1 once it is mounted to the drive shaft 1. An annular groove 19b on the end section 19 may be provided that cooperates with a corresponding projection in the handle 5 to prevent inadvertent disassembling of the handle.

By means of the cylindrical drive shaft 1 a longitudinal axis L of the instrument is defined.

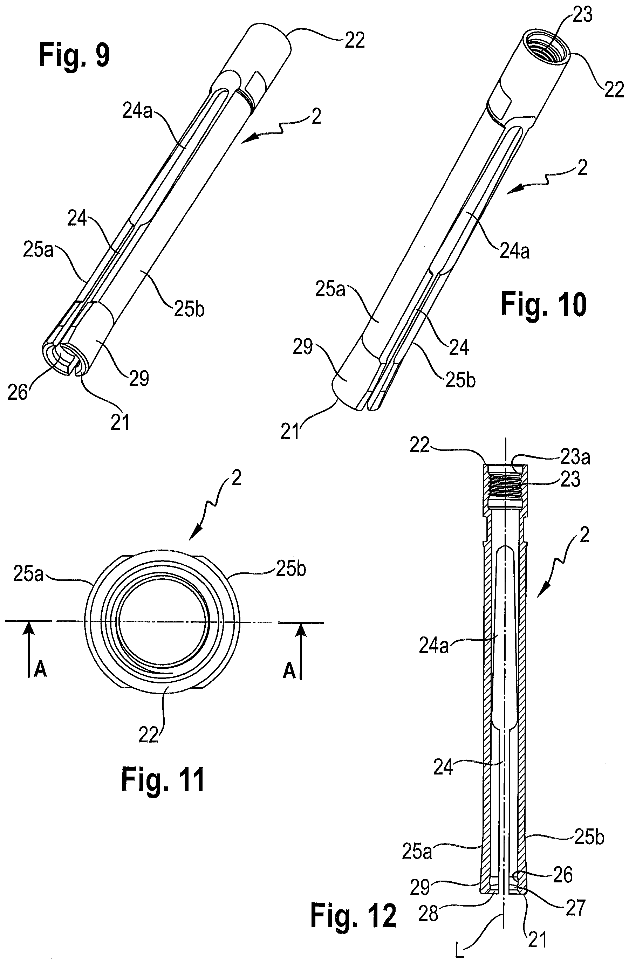

Next, the holding member 2 will be explained referring to Figs. 9 to 12. The holding member 2 is sleeve-shaped and has a first or proximal end 21 and an opposite second or distal end 22. At a distance from the second end 22 there is an internally threaded section 23 with a slightly smaller diameter than that of the sleeve at the second end 22. The internally threaded section 23 cooperates with the threaded section 15 on the drive shaft 1. By the reduction of the diameter a shoulder 23a is formed that faces towards the second end 22 and that cooperates with the portion 16b on the drive shaft 1. The holding member is slotted by a slot 24 that is open to the first end 21 and that extends through the holding member 2 in a plane including the longitudinal axis L so that two resilient arms 25a, 25b are formed by the slot 24. The slot 24 widens into a substantially elongate opening 24a and is that closed towards the second end 22. The opening 24a provides greater flexibility to the arms 25a, 25b.

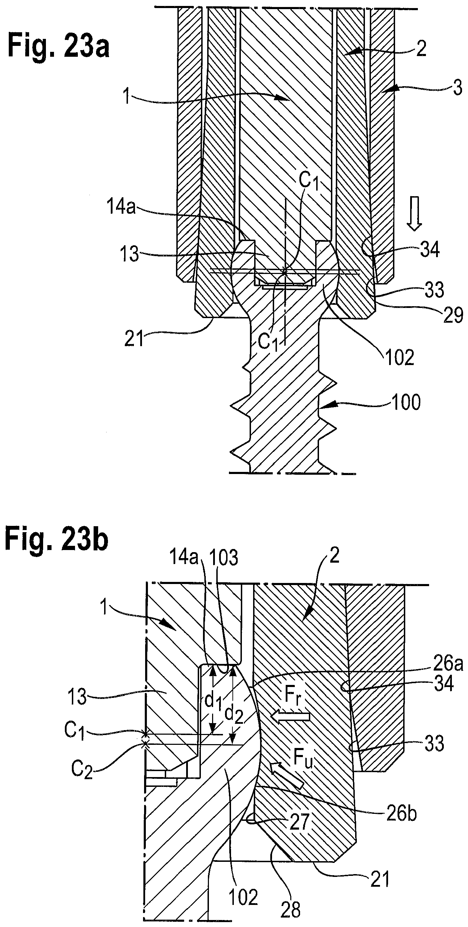

At a distance from the first end 21, there is an inner spherical segment-shaped section that provides a seat 26 for the head 102 of the bone anchor. The radius of the inner spherical segment-shaped section 26 is such that it substantially matches the shape of an outer surface portion of the head 102. As can be seen more in detail in Fig. 23b, the spherical segment-shaped section 26 is designed such that a region with a largest diameter of the sphere defined by the section 26 is between an upper edge 26a and a lower edge 26b of the seat. Hence, when the head 102 is received in the seat 26 and the arms 25a, 25b are pressed together, the head 102 is held in a form-fit manner in the seat 26. Adjacent to the seat 26, there is a cylindrical section 27 with an inner diameter that is smaller than that of the lower edge 26b of the seat 26. Between the cylindrical section 27 and the first end 21, there is a tapered section 28 that tapers towards the seat defined by the seat 26. When the head 102 is received in the seat 26 and the arms 25a, 25b are pressed together, the head 102 is held in the holding member such that it can not be removed through the first end 21.

Referring more in detail to Figs. 6 and 23b, the center of the sphere C1 of the seat 26 is offset in an axial direction from the center of the sphere C₂ of the head 102 when the head 102 is placed into the seat 26. More specifically, in the second configuration, the distance di of the center of the sphere of the seat 26 from the abutment surface 14a of the drive shaft is smaller than the distance d2 of the center of the sphere C2 of the head 102 from the abutment surface 14a when the head 102 is held in the seat 26 and the two arms 25a, 25b are pressed together. By means of this, when the head 102 is held in the seat 26 it experiences a force that tends to move the head 102 towards the abutment surface 14a.

The dimensions of the lower portion of the holding member that includes the seat 26, the cylindrical portion 27 and the tapered portion 28 correspond substantially to a receiving part of a polyaxial bone screw or are slightly larger in an axial direction. Therefore, when the head 3 is held in the holding member 2 and inserted into the bone, the first end 21 of the holding member forms an abutment for the bone surface so that the bone anchor 100 is inhibited from further insertion into the bone. The head 102 protrudes above the bone surface to such an extent that the receiving part can be easily mounted thereon.

The length of the holding member 2 is such that when the holding member 2 is screwed onto the threaded portion 15 of the drive shaft until its second end 22 abuts against the abutment surface 16a, the first end 21 extends beyond the first end 11 of the drive shaft by a distance. The distance is such that the offset between the centers of the spheres C₁ and C₂ of the seat 26 and the head 102, respectively, causes the free end surface 103 of the head 102 to be pressed against the abutment surface 14a of the drive shaft 1. An inner diameter of the holding member 2 is such that the portion 14 of the drive shaft can pass there through and is guided therein.

Furthermore, as depicted in particular in Fig. 12, an outer surface portion 29 adjacent to the first end 21 of the holding member 2 is tapered such that it narrows towards the second end 22. Hence, the holding member 2 resembles a collet chuck and functions like a collet chuck.

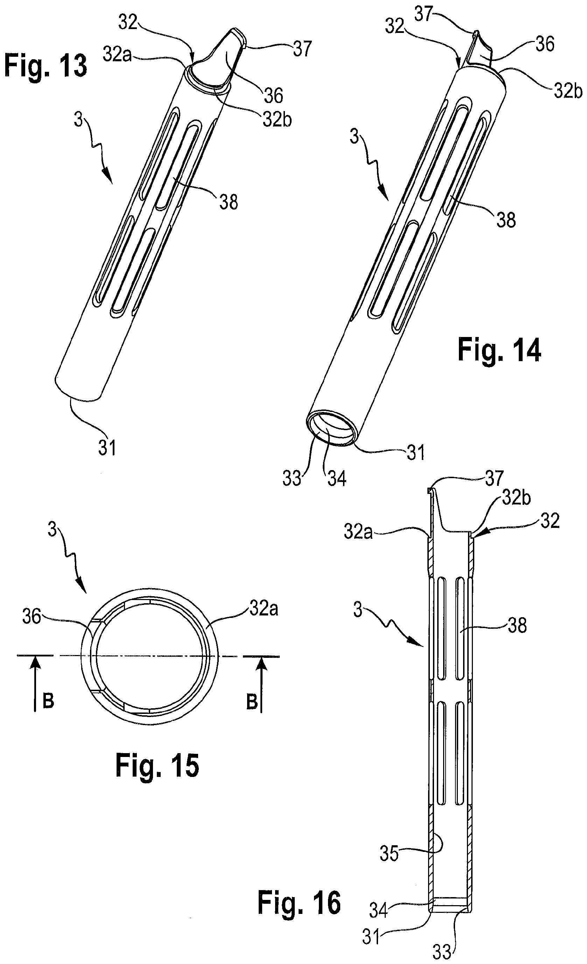

The displacement member 3 will be described with reference to Figs. 4 to 6 and 13 to 16. The displacement member 3 is formed as a substantially cylindrical sleeve with a first or proximal end 31 and an opposite second or distal end 32 of the sleeve. An inner diameter of the displacement member 3 is such that, as can be seen in Figs. 4 to 6, the holding member 2 can extend through the displacement member 3. Adjacent to the first end 31 an inner surface 33 of the displacement member is slightly tapered in a direction such that it widens towards the first end 31. This slightly tapered section 33 is followed towards the second end by a second slightly tapered section 34 with an angle of the taper that is even smaller than the angle of the taper of the section 33. The remaining part of the displacement member 3 has a cylindrical inner surface 35. The function of the slightly tapered surfaces 34 and 33 is to cooperate with the tapered outer surface 29 of the holding member 2 when the displacement member 3 is moved from its first position in the first configuration to the second position in the second configuration. During this displacement, the tapered inner surfaces 34 and 33 slide along the tapered outer surface 29 thereby generating an increasing pressure onto the arms 25a, 25b that presses the arms together to clamp the inserted head 102.

The second end 32 has a free annular end surface 32a that acts as an abutment for the actuator 4 as described below. From the end surface 32a, a short circular rim 32b with a smaller inner diameter protrudes upward. Moreover, a flap-like extension or flap 36 projects above the rim 32b. The flap 36 has a substantially cylindrical inner surface and a substantially cylindrical outer surface and a trapezoidal contour when viewed in a side front view, hence, its width on the basis adjacent to the rim 32b is larger than its free end. Furthermore, at the free end, the flap 36 comprises an outwardly extending circumferential projection 37 that cooperates with a portion of the actuator 4 to be described below. The height of the flap 36 between the shoulder 32a and the outward projection 37 corresponds substantially to the height of an inner portion of the actuator 4 in an axial direction. The flap 36 has the function of a follower that rotatably connects the displacement member 3 to the actuator but prevents an axial movement of the displacement member 3 relative to the actuator 4. An axial advancement of the actuator results in a following axial advancement of the displacement member 3.

The displacement member 3 may further have a plurality of elongated openings 38 that extend completely through the cylindrical wall portion. The openings 38 serve for facilitating the cleaning of the parts.

A total axial length of the displacement member 3 is such that in a position of the actuator 4 that corresponds to the first configuration, the first end 31 has a distance from the first end 21, of the holding member that allows the holding member 2 to spread outward from the first end 31 of the displacement member 3 to allow the insertion of the head. Furthermore, the length is such that in a second position of the actuator 4 that corresponds to the second configuration, the tapered inner sections 33, 34 cooperate with the tapered outer surface 29 of the holding member to clamp the head 102.

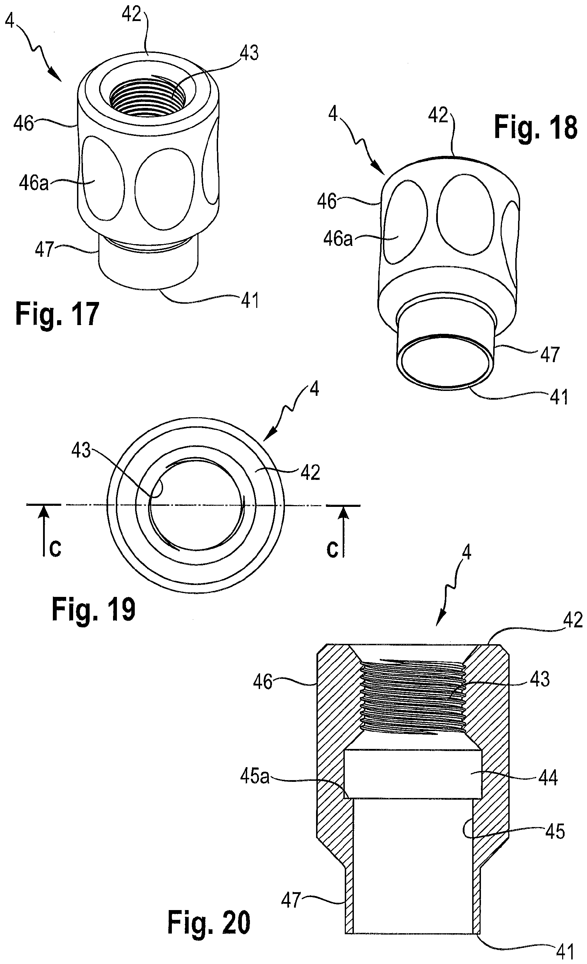

The actuator 4 will be described with reference to Figs. 4 to 6 and 17 to 20. The actuator 4 comprises a first end or proximal end 41 and an opposite second or distal end 42. At a small distance from the second end 42 a bore 43 with an internal thread is provided that cooperates with the external thread of the section 18 of the drive shaft 1. Therefore, the internal thread is also preferably a multi-start fine thread. Adjacent to the threaded bore 43, there is an inner substantially cylindrical section 44 that has a diameter greater than an outer diameter of the threaded section 18 of the drive shaft 1 such that the drive shaft 1 can extend therethrough with the threaded section 18. Between the cylindrical inner section 44 and the first end 41, there is a second cylindrical section 45 with a smaller diameter than the section 44 such that an annular shoulder 45a is provided within the actuator 4. An inner diameter of the cylindrical section 45 is such that the portion 17 of the drive shaft 1 and in addition the flap 36 of the displacement member 3 can extend therethrough, as can be seen in particular in Fig. 5. A distance between the shoulder 45a and the first end 41 is such that the flap 36 can abut with the lower side of its circumferential outward projection 37 against the shoulder 45a and the first end 41 of the actuator can abut against the free end surface 32a of the displacement member 3. Hence, the displacement member 3 is configured to be connected via the flap 36 to the actuator 4 such that the actuator 4 can rotate with respect to the displacement member 3 but the displacement member 3 is fixed in an axial direction with respect to the actuator 4.

The actuator 4 further comprises an upper substantially cylindrical outer section 46 adjacent to the second end 42 with a plurality of flats 46a that facilitate gripping. The flats 46a are shown to be oval-shaped, but can have any other shape. The number can be any number. Instead of the flats 46a other means can be used for facilitate gripping, such as, for example, longitudinal ripples or grooves or a roughened surface.

The actuator further comprises a second outer cylindrical section 47 adjacent to the first end 41 with an outer diameter that substantially corresponds to an outer diameter of the displacement member 3 so that the actuator 4 is flush with the displacement member 3 as depicted in Figs. 3 to 5.

Referring further to Fig. 1, the handle 5 comprises a coaxial bore 51 that has an internal flat portion that cooperates with the flat 19a of the drive shaft, so that the handle 5 can be mounted onto the rear end portion 19 of the drive shaft 1 in a rotationally fixed manner. The bore 51 of the handle 5 may have an internal annular projection that engages the groove 19b of the drive shaft 1 so that the handle 5 may be clicked onto the drive shaft 1.

All parts of the instrument that may have contact with tissue or bone may be made of a body-compatible material, such as, for example, stainless steel.

The instrument is easy to assemble and disassemble. This provides the advantage that after use it can be easily cleaned. The instrument is mounted as follows. First the holding member 2 is screwed onto the drive shaft so that the first portion 14 of the drive shaft extends through the holding member 2. The position of the holding member 2 on the drive shaft is limited in an axial direction by the abutment of the second end 22 of the holding member against the abutment surface 16a. Then the displacement member 3 is connected to the actuator 4 in that the flap 36 is inserted into the actuator 4 until its projection 37 rests on the shoulder 45a and the first end 41 of the actuator rests on the free end surface 32a of the displacement member 3. Then, the pre-assembled displacement member 3 and actuator 4 are placed onto the drive shaft 1 from the second end 12 of the drive shaft so that the internal threaded portion 43 of the actuator is screwed onto the externally threaded portion 18 of the drive shaft 1. Finally, the handle 5 is clicked onto the rearward end 19 of the drive shaft 1.

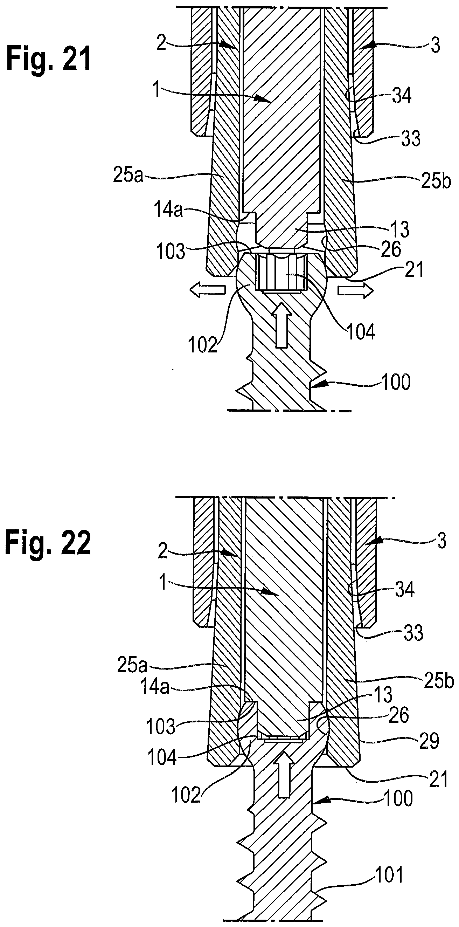

The use of the instrument with a bone anchor will be described with reference to Figs. 21 to 23b. In the first configuration, the actuator 4 is in a first position closer to the second end 12 of the drive shaft 1 than in the second configuration. As shown in Fig. 2, to reach the first configuration which is the "open configuration", the actuator 4 is rotated by hands in the counter clockwise direction, so that the displacement member 3 follows this movement and assumes a retracted position. The arms 25a, 25b of the holding member project out of the displacement member 3 to such an extent that they are configured to be resiliently pressed apart from each other. As depicted in Fig. 21, in the first configuration, the head 102 presses the two arms 25a, 25b away until it snaps into the seat 26. During the insertion, the engagement projection 13 of the drive shaft enters into a corresponding engagement recess 104 provided at the free end surface of the head 102. The engagement projection 13 can enter into the recess 104 until the free end surface 103 of the head 102 abuts against the abutment surface 14a of the drive shaft. During this insertion step, the shank 101 is aligned with the drive shaft 1. The head 102 is held in a form-fit manner in the axial direction and also in the seat 26 so that it cannot be removed easily.

By rotating the actuator 4 in the clockwise direction, the displacement member 3 is moved towards the first end of the holding member 2 as depicted in Fig. 3. When the displacement member 3 moves toward the first end 21 of the holding member 2, the tapered inner surfaces 33, 34 of the displacement member 3 come into contact with the outer tapered surface portion 29 of the holding member 2 as depicted in Fig. 23a. Thereby, the two arms 25a, 25b are pressed together. Simultaneously, the pressure is transmitted to the head 102 as depicted through the horizontal arrow Fᵣ in Fig. 23b. Because the center of the sphere C₁ of the spherical seat 26 is offset with respect to the center of the sphere C2 of the head 102 in a direction toward the abutment surface 14a of the drive shaft 1, a force Fᵤ acts onto the head 102 that has a component directed in an axial direction towards the abutment surface 14a. As a result thereof the head 102 is firmly pressed onto the abutment surface 14a. A possible play between the engagement portion 13 and the engagement recess 104 is eliminated. Therefore, the surgeon experiences an insertion of the screw without any play.

In the second configuration which is the "closed configuration", the head 102 is firmly held in the holding member. In this configuration it can be inserted into the bone. The first end 21 of the holding member 2 serves as an abutment for the bone that limits the insertion and ensures that a sufficient portion of the bone anchor projects over the bone surface so that the receiving part can be mounted onto the head 102 afterwards.

An embodiment of a system of the instrument and a bone anchor comprises the above described instrument and a bone anchor having a head, wherein the seat and the dimensions of the holding member is adapted to the dimension of the head.

Modifications of the above described embodiments are possible.

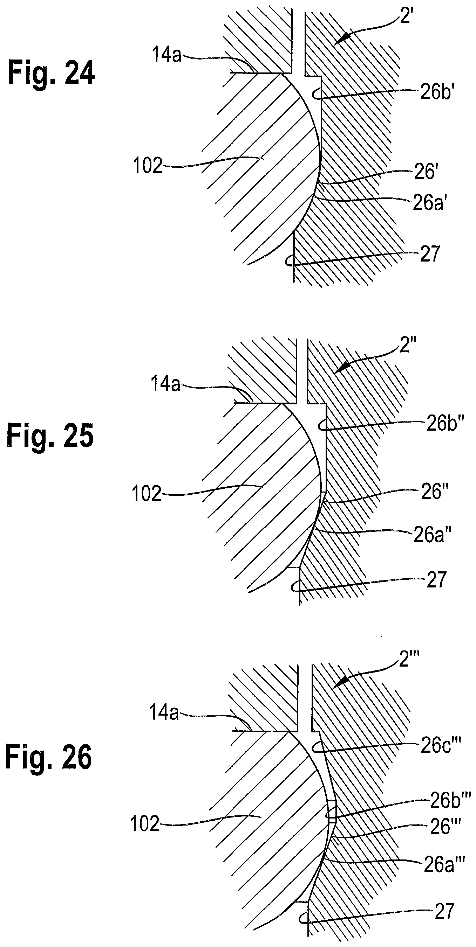

Referring to Figs. 24 to 26, the seat for the head in the holding member 2 can be designed in a different shape. Parts and portions that are identical to the previously described embodiment are marked with the same reference signs. For example, as depicted in Fig. 24, the holding member 2' may have a seat 26' that has a spherical segment shape 26a' adjacent to the lower cylindrical portion which continues in a slightly conically widening portion 26b' in a direction towards the second or distal end. As in the previous embodiment, the size of the seat portions is such that when the arms are compressed, the head experiences a force that presses it towards the abutment surface 14a.

In the embodiment shown in Fig. 25, the seat 26" has a lower tapered portion 26a" that tapers towards the first or proximal end and continues in a cylindrical portion 26b" towards the second end. The inner diameter of the cylindrical portion is slightly larger than the largest diameter of the head 102, so that when the arms are pressed together the tapered portion exerts a force onto the head that tries to move the head upward, however because of the abutment of the head against the abutment surface 14a, the head is firmly pressed against the abutment surface 14a.

In the embodiment shown in Fig. 26, the seat comprises a first tapered portion 26a'" that is tapered towards the first end, followed by a cylindrical portion 26b'" which is followed by a second tapered portion 26c'" that tapers towards the second end. The function is the same as for the other embodiments.

In all embodiments the seat has such a size that when the arms are compressed, the head experiences a force that moves it upward in the direction of the abutment surface whereby it is pressed against the abutment surface.

As a further example for a modification, the actuator can be realized by other means that allow to displace the displacement member to obtain the first and the second configuration.

The actuator can also be omitted. For example, the displacement member may have an internal thread that cooperates with an external thread of the holding member and allows an advancement of the displacement member along the holding member.

The abutment surface needs not to be circular or ring-shaped. Any abutment surface can be used that provides sufficient area to generate enough friction to eliminate the play between the engagement projection 13 and the engagement recess 104 of the head.

The holding member can have more than two arms, for example three or more arms that can be pressed together in a resilient manner.

Instead of the threaded connections shown in the embodiment, other detachable connections can be provided.

For the bone anchor, any bone anchor can be used that has a shank and a head portion. It is not necessary that the bone anchor has to be screwed in. The instrument is also suitable for pushing a bone anchor into a hole.

While the head of the bone anchor and the seat are shown to be designed for a polyaxial bone anchor, it is also possible that the head and the seat have flat side portions that render the bone anchor suitable for a monoplanar bone anchoring device that allows pivoting only in a single plane.

## Claims

1. A system comprising:
a bone anchor (100) comprising a shank (101) for anchoring in the bone and a head (102), the bone anchor in particular being a pedicle screw; and
an instrument for holding and inserting the bone anchor into the bone; the instrument comprising
a holding member (2) for holding the head (102) of the bone anchor, the holding member (2) comprising at least two arms (25a, 25b) that are configured to at least partially encompass the head (102) of the bone anchor and wherein the at least two arms (25a, 25b) comprise an inner surface forming a seat (26) for the head;
a drive shaft (1) for engaging the head (102) of the bone anchor, the drive shaft (1) defining a longitudinal axis (L) of the instrument; and
a displacement member (3) that is configured to act onto the holding member (2) such that the holding member (2) can assume a first configuration in which it is configured to permit the head to enter the seat (26) and a second configuration in which it is configured to hold the head in the seat (26);
wherein the drive shaft (1) comprises an abutment surface (14a) for the head,
**characterized in that** the seat (26) has a lower tapered portion (26a", 26a'") that tapers towards a first end (21) of the holding member (2), and
the seat (26) is provided at such an axial position of the holding member (2) relative to the drive shaft (1), that when the two arms (25a, 25b) are pressed together, the tapered portion (26a", 26a"') exerts a force onto the head that moves an inserted head (102) upward toward a second end (22) of the holding member (2) to press the same against the abutment surface (14a) in the second configuration.

2. The system of claim 1, wherein the head (102) has an spherical outer surface portion and the seat (26) comprises a matching spherical inner surface portion.

3. The system of claim 2, wherein a region of largest inner diameter of the spherical inner surface portion of the seat (26) is situated between an upper edge (26a) and a lower edge (26b).

4. The system of claim 2 or 3, wherein the center (C₁) of the sphere that forms the spherical inner surface portion of the seat (26) is closer to the abutment surface (14a) than the center (C₂) of the sphere that forms the spherical outer surface of the head (102), wherein the center (C₁) is offset in the axial direction from the center (C₂) when the head is placed into the seat.

5. The system of one of claims 1 to 4, wherein the drive shaft (1) has a free end (11) that comprises an engagement portion (13) and wherein the abutment surface (14a) is provided at a distance from the free end and is separate from the engagement portion (13).

6. The system of one of claims 1 to 5, wherein the holding member (2) comprises a first sleeve with a first end (21) with at least one slot (24) such that the arms are formed by the slot (24) and wherein the arms (25a, 25b) are resilient towards each other.

7. The system of claim 6, wherein the holding member (2) comprises a second end (22) and wherein the drive shaft (1) extends through the sleeve and is connected to the holding member at the second end (22).

8. The system of claim 6 or 7, wherein the displacement member (3) comprises a second sleeve that is arranged around at least a portion of the first sleeve and that is displaceable relative the first sleeve in a longitudinal direction.

9. The system of one of claims 1 to 8, further comprising an actuator (4) for actuating the displacement member (3) and for holding the displacement member (3) relative to the holding member (2) in the first configuration and in the second configuration.

10. The system of claim 9, wherein the actuator (4) comprises a third sleeve that is connected to the drive shaft via a threaded connection (15, 18), preferably a multi-start thread.

11. The system of claim 9 or 10, wherein the displacement member (3) is connected to the actuator (4) such that a movement of the actuator in a longitudinal direction displaces the displacement member in the longitudinal direction.

12. The system of one of claims 1 to 11, wherein the holding member (2) is detachably connected to the drive shaft (1).

13. The system of one of claims 9 to 12, wherein the actuator is detachably connected to the drive shaft (1) and is further preferably detachably connected to the displacement member (3).

14. The system of one of claims 1 to 13, wherein the dimension of the head (102) is adapted to the dimension of the seat (26).

15. The system of claim 14, wherein the head (102) has a spherical outer surface portion.

## Patentansprüche

1. Ein System, umfassend:
einen Knochenanker (100) umfassend einen Schaft (101) zum Verankern in dem Knochen und einen Kopf (102), wobei der Knochenanker insbesondere eine Pedikelschraube ist; und
ein Instrument zum Halten und Einsetzen des Knochenankers in den Knochen, wobei das Instrument umfasst:
ein Halteglied (2) zum Halten des Kopfs (102) des Knochenankers, wobei das Halteglied (2) wenigstens zwei Arme (25a, 25b) umfasst, die eingerichtet sind, zumindest teilweise den Kopf (102) des Knochenankers zu umgreifen, wobei die wenigstens zwei Arme (25a, 25b) eine Innenoberfläche umfassen, die einen Sitz (26) für den Kopf ausbildet;
einen Antriebsschaft (1), der in den Kopf (102) des Knochenankers eingreift, wobei der Antriebsschaft (1) eine Längsachse (L) des Instruments festlegt; und
ein Verschiebungsglied (3), das eingerichtet ist, so auf das Halteglied (2) einzuwirken, dass das Halteglied (2) eine erste Konfiguration einnehmen kann, in welcher es eingerichtet ist, dem Kopf zu erlauben, im Sitz (26) platziert zu werden, und eine zweite Konfiguration einnehmen kann, in welcher es eingerichtet ist, den Kopf in dem Sitz (26) zu halten;
wobei der Antriebsschaft (1) eine Anschlagoberfläche (14a) für den Kopf umfasst,
**dadurch gekennzeichnet, dass** der Sitz (26) einen unteren, sich verjüngenden Abschnitt (26a", 26a'") besitzt, der sich in Richtung auf das erste Ende (21) des Halteglieds (2) verjüngt, und
der Sitz (26) an einer solchen axialen Position des Halteglieds (2) relativ zum Antriebsschaft (1) vorgesehen ist, dass, wenn die zwei Arme (25a, 25b) zusammengedrückt werden, der sich verjüngende Abschnitt (26a", 26a'") eine Kraft auf den Kopf ausübt, die einen eingesetzten Kopf (102) nach oben in Richtung auf das zweite Ende (22) des Haltglieds (2) bewegt, um denselben gegen die Anschlagoberfläche (14a) in der zweiten Konfiguration zu drücken.

2. Das System gemäß Anspruch 1, wobei der Kopf (102) des Knochenankers (100) einen sphärischen Außenoberflächenabschnitt besitzt und der Sitz (26) einen dazu passenden sphärischen Innenoberflächenabschnitt umfasst.

3. Das System gemäß Anspruch 2, wobei ein Gebiet größten Innendurchmessers des sphärischen Innenoberflächenabschnitts des Sitzes (26) zwischen einer oberen Kante (26a) und einer unteren Kante (26b) positioniert ist.

4. Das System gemäß Anspruch 2 oder 3, wobei der Mittelpunkt (C₁) der Sphäre, die den sphärischen Innenoberflächenabschnitt des Sitzes (26) ausbildet, näher an der Anschlagoberfläche (14a) ist als der Mittelpunkt (C₂) der Sphäre, die den sphärischen Außenoberflächenabschnitt des Kopfs (102) ausbildet, wobei der Mittelpunkt (C₁) in der axialen Richtung gegenüber dem Mittelpunkt (C₂) versetzt ist, wenn der Kopf in dem Sitz platziert ist.

5. Das System gemäß einem der Ansprüche 1 bis 4, wobei der Antriebsschaft (1) ein freies Ende (11) besitzt, das einen Eingriffsabschnitt (13) aufweist, und wobei die Anschlagoberfläche (14a) in einem Abstand von dem freien Ende vorgesehen und getrennt von dem Eingriffsabschnitt (13) ist.

6. Das System gemäß einem der Ansprüche 1 bis 5, wobei das Halteglied (2) eine erste Hülse mit einem ersten Ende (21) mit wenigstens einem Schlitz (24) umfasst, so dass die Arme durch den Schlitz (24) gebildet werden, und wobei die Arme (25a, 25b) in Richtung zueinander federnd ausgebildet sind.

7. Das System gemäß Anspruch 6, wobei das Halteglied (2) ein zweites Ende (22) umfasst, und wobei sich der Antriebsschaft (1) durch die Hülse erstreckt und mit dem Halteglied an dem zweiten Ende (22) verbunden ist.

8. Das System gemäß Anspruch 6 oder 7, wobei das Verschiebungsglied (3) eine zweite Hülse umfasst, die um zumindest einen Abschnitt der ersten Hülse herum angeordnet ist, und welche relativ zu der ersten Hülse in einer Längsrichtung verschiebbar ist.

9. Das System gemäß einem der Ansprüche 1 bis 8, ferner umfassend einen Aktuator (4) zum Betätigen des Verschiebungsglieds (3) und zum Halten des Verschiebungsglieds (3) relativ zu dem Halteglied (2) in der ersten Konfiguration und in der zweiten Konfiguration.

10. Das System gemäß Anspruch 9, wobei der Aktuator (4) eine dritte Hülse umfasst, die mit dem Antriebsschaft über eine Gewindeverbindung (18) verbunden ist, vorzugsweise ein mehrgängiges Gewinde.

11. Das System gemäß Anspruch 9 oder 10, wobei das Verschiebungsglied (3) mit dem Aktuator (4) verbunden ist, so dass eine Bewegung des Aktuators in einer Längsrichtung das Verschiebungsglied in der Längsrichtung verschiebt.

12. Das System gemäß einem der Ansprüche 1 bis 11, wobei das Halteglied (2) lösbar mit dem Antriebsschaft (1) verbunden ist.

13. Das System gemäß einem der Ansprüche 9 bis 12, wobei der Aktuator lösbar mit dem Antriebsschaft (1) verbunden ist und ferner vorzugsweise mit dem Verschiebungslied (3) lösbar verbunden ist.

14. Das System gemäß einem der Ansprüche 1 bis 13, wobei die Größe des Kopfs (102) des Knochenankers an die Größe des Sitzes (26) angepasst ist.

15. Das System gemäß Anspruch 14, wobei der Kopf (102) einen sphärischen Außenoberflächenabschnitt besitzt.

## Revendications

1. Un système comprenant :
une ancre osseuse (100) comprenant une tige (101) pour l'ancrage dans l'os et une tête (102), l'ancre osseuse en particulier étant une vis pédiculaire, et
un instrument pour maintenir et insérer l'ancre osseuse dans l'os, l'instrument comprenant
un élément de maintien (2) pour maintenir la tête (102) de l'ancre osseuse, l'élément de maintien (2) comprenant au moins deux bras (25a, 25b) qui sont configurés pour entourer au moins partiellement la tête (102) de l'ancre osseuse et dans lequel les au moins deux bras (25a, 25b) comprennent une surface interne formant un siège (26) pour la tête,
un arbre d'entraînement (1) pour engager la tête (102) de l'ancre osseuse, l'arbre d'entraînement (1) définissant un axe longitudinal (L) de l'instrument, et
un élément de déplacement (3) qui est configuré pour agir sur l'élément de maintien (2) de telle sorte que l'élément de maintien (2) peut adopter une première configuration dans laquelle il est configuré pour permettre à la tête d'entrer dans le siège (26) et une seconde configuration dans laquelle il est configuré pour maintenir la tête dans le siège (26),
dans laquelle l'arbre d'entraînement (1) comprend une surface de butée (14a) pour la tête,
**caractérisé en ce que** le siège (26) a une partie inférieure effilée (26a", 26a"') qui s'effile vers une première extrémité (21) de l'élément de maintien (2), et
le siège (26) est prévu dans une position axiale de l'élément de maintien (2) par rapport à l'arbre d'entraînement (1) telle que, lorsque les deux bras (25a, 25b) sont pressés ensemble, la partie effilée (26a', 26a"') exerce sur la tête une force qui déplace une tête (102) introduite vers le haut vers une seconde extrémité (22) de l'élément de maintien (2) pour appuyer celle-ci sur la surface de butée (14a) dans la seconde configuration.

2. Le système selon la revendication 1, dans lequel la tête (102) présente une partie de surface extérieure sphérique et le siège (26) comprend une partie de surface intérieure sphérique assortie.

3. Le système selon la revendication 2, dans lequel une région du plus grand diamètre intérieur de la partie de surface intérieure sphérique du siège (26) est située entre un bord supérieur (26a) et un bord inférieur (26b).

4. Le système selon la revendication 2 ou 3, dans lequel le centre (C₁) de la sphère qui forme la partie de surface intérieure sphérique du siège (26) est plus proche de la surface de butée (14a) que le centre (C₂) de la sphère qui forme la surface extérieure sphérique de la tête (102), le centre (C₁) étant décalé dans la direction axiale du centre (C₂) lorsque la tête est placée sur le siège.

5. Le système selon l'une des revendications 1 à 4, dans lequel l'arbre d'entraînement (1) présente une extrémité libre (11) qui comprend une partie d'engagement (13) et dans lequel la surface de butée (14a) est prévue à une distance de l'extrémité libre et est distincte de la partie d'engagement (13).

6. Le système selon l'une des revendications 1 à 5, dans lequel l'élément de maintien (2) comprend un premier manchon avec une première extrémité (21) avec au moins une fente (24) de sorte que les bras sont formés par la fente (24) et dans lequel les bras (25a, 25b) sont élastiques vers chaque autre.

7. Le système selon la revendication 6, dans lequel l'élément de maintien (2) comprend une deuxième extrémité (22) et dans lequel l'arbre d'entraînement (1) traverse le manchon et est relié à l'élément de retenue à la deuxième extrémité (22).

8. Le système selon l'une des revendications 6 ou 7, dans lequel l'élément de déplacement (3) comprend un deuxième manchon qui est disposé autour d'au moins une partie du premier manchon et qui peut être déplacé par rapport au premier manchon dans une direction longitudinale.

9. Le système selon l'une des revendications 1 à 8, comprenant en outre un actionneur (4) pour actionner l'élément de déplacement (3) et pour maintenir l'élément de déplacement (3) par rapport à l'élément de maintien (2) dans la première configuration et dans la seconde configuration.

10. Le système selon la revendication 9, dans lequel l'actionneur (4) comprend une troisième manchon qui est reliée à l'arbre d'entraînement par l'intermédiaire d'un raccord fileté (15, 18), de préférence un filetage à démarrage multiple.

11. Le système selon l'une des revendications 9 ou 10, dans lequel l'élément de déplacement (3) est relié à l'actionneur (4) de telle sorte qu'un mouvement de l'actionneur dans une direction longitudinale déplace l'élément de déplacement dans la direction longitudinale.

12. Le système selon l'une des revendications 1 à 11, dans lequel l'élément de maintien (2) est relié de manière amovible à l'arbre d'entraînement (1).

13. Le système selon l'une des revendications 9 à 12, dans lequel l'actionneur est relié de manière amovible à l'arbre d'entraînement (1) et est en outre de préférence relié de manière amovible à l'élément de déplacement (3).

14. Le système selon l'une des revendications 1 à 13, dans lequel la dimension de la tête (102) est adaptée à la dimension du siège (26).

15. Le système selon la revendication 14, dans lequel la tête (102) présente une partie de surface extérieure sphérique.
